# EUROPEAN PATENT SPECIFICATION

(11) **EP 1 263 413 B1**
(45) Date of publication and mention of the grant of the patent: **24.05.2006**
(21) Application number: 01914278.5
(22) Date of filing: 08.03.2001
(51) Int. Cl.: A61K 9/14, A61K 31/165

(54) **METHOD OF PREPARING SOLID DISPERSIONS**
VERFAHREN ZUR HERSTELLUNG FESTER DISPERSIONEN
PROCEDE DE PREPARATION DE DISPERSIONS SOLIDES

(30) Priority: 09.03.2000 US 187984 P; 21.03.2000 SE 0000934
(43) Date of publication of application: 11.12.2002
(73) Proprietor: Ohio State University Research Foundation, Columbus, OH 43210-1063 (US)
(72) Inventor: BRODIN, Arne, S-151 54 Södertälje (SE); FRANK, Sylvan, Columbus, OH 43212-1034 (US); YE, Chao, Waukegan, IL 60085 (US)
(74) Representative: Mercer, Christopher Paul
(86) International application number: PCT/SE2001/000499
(87) International publication number: WO 2001/066091

(56) References cited:
- WO-A2-99/65469
- J. KERC ET AL.: 'Micronization of drugs using supercritical carbon dioxide' INTERNATIONAL JOURNAL OF PHARMACEUTICS vol. 182, 1999, pages 33 - 39, XP001037772

## Description

The present invention is directed to improved methods for producing solid dispersions, especially pharmaceutical dispersions. These methods involve the dissolution of one or more insoluble or relatively insoluble substances, preferably pharmaceutically active substances, and/or one or more water-soluble substances, preferably carriers, in supercritical fluids and the subsequent removal of solvent by rapid expansion.

### Background of the Invention

By increasing surface area, reducing particle size tends to enhance the rate of dissolution of insoluble and relatively insoluble substances, like pharmaceuticals. Thus, in general, particles should be as small as possible. Substances that are sparingly soluble in water, like pharmaceuticals often provide sustained therapeutic relief and good tissue partitioning. Unfortunately, these pharmaceuticals usually exhibit poor bioavailability and, as a result, have a slow onset of action. One method for increasing the bioavailability of insoluble and relatively soluble pharmaceuticals is to formulate them into a solid dispersion of small particles.

Traditionally, solid dispersions of small particle preparations, like pharmaceuticals, have been formed by one of three methods: the fusion method; the solvent method; and the fusion-solvent method. In the fusion method, a physical mixture of the components is heated until it is melted, the melt cooled and the resulting solid pulverized and sieved. This method is suitable for pharmaceuticals and carriers that are miscible in the molten state and for which melting is easily achieved. The disadvantages of the method are thermal degradation of the pharmaceutical, sublimation of the pharmaceutical and/or carrier and thermally induced transformation of the carrier. In addition, the hardening of the melt is sometimes difficult to achieve and may result in tacky, unmanageable solid dispersions.

In the solvent method, the preparation components are dissolved in a common solvent and precipitation of solutes is induced by the removal of solvent or by the addition of an anti-solvent. Compared with the fusion method, the solvent method is more likely to produce solid solutions or amorphous precipitates because both components, like pharmaceutical and carrier, are molecularly dispersed in the solvent. The disadvantages of the method are the difficulty in finding a suitable non-toxic co-solvent since carriers are usually hydrophilic whereas pharmaceuticals are usually hydrophobic, the generally low evaporative rates of organic solvents even under reduced pressure, the difficulty of completely removing organic solvents and the costs and hazards involved in handling large volumes of organic solutions.

The fusion-solvent method is a combination of the first two methods. The substance e.g a pharmaceutical is dissolved in a suitable solvent and the solution, usually containing 5-10% W/W of pharmaceutical, is incorporated into the melted carrier. This method is generally suitable for thermo-labile pharmaceuticals, but only small amounts of substance, like the pharmaceutical, can be incorporated and the melting point of the carrier has to be low enough to avoid thermal degradation. Again, the complete removal of solvent may be difficult or impossible.

More recently, a technique has been developed for obtaining solid dispersions based upon the use of supercritical fluids. These fluids are highly compressed gases which, when under pressure, may be used to dissolve pharmaceuticals. Solid dispersions may then be produced by the rapid expansion of the supercritical solution (RESS) and the resulting transformation of solvent into the gas phase. In a typical RESS procedure, powders, like pharmaceutical powders, would be dissolved in a fluid such as supercritical carbon dioxide (SCCO₂). The supercritical solution thus formed is allowed to pass through a small nozzle or capillary tube and to then expand under atmospheric pressure and temperature. As the SCCO₂ converts to a gas, fine pharmaceutical particles precipitate and CO₂ is then quantitatively removed from the system. The rapid, and essentially explosive, expansion causes a substantial decrease of solvent power, *i*.*e*., up to several orders of magnitude and, as a result, a high degree of super saturation is achieved within an extremely short period of time. Simultaneously, the shock wave induced by the pressure change propagates at a supersonic rate and instantaneously provides numerous nuclei for crystal growth. This results in the formation of a uniform distribution of very small particles.

The RESS procedure has a number of advantages over traditional methods for forming compositions, preferably pharmaceutical compositions. First, organic solvents are not involved and, as a result, the process is relatively safe and environmentally clean. Supercritical solvent can be easily removed by expansion since CO₂ is a gas under ambient conditions and the solvent can be easily recovered for use in the future. The process produces uniformly distributed particles and is easily automated since temperature and pressure are the only parameters that need to be controlled. Finally, the process is economically favourable because agitation, precipitation and solvent removal occur simultaneously and there is no need for special procedures to deal with the handling and disposal of hazardous material.

Despite the advantages of RESS preparation, the identification of combinations of substances that are compatible with the method remains a challenge. This is especially true in cases where a composition will contain both a hydrophobic substance and a hydrophilic substance. Particularly, in cases with a pharmaceutical formulation, where a hydrophobic pharmaceutical and a hydrophilic carrier are involved. In addition, formulations are needed which are capable of providing a therapeutic agent for the treatment of a condition both rapidly and over a period of time.

Alternative methods are described in e.g., 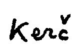 et al, Int. J. Pharmaceutics 1999; 182 : 33-39 and Subramaniam et al, J. Pharm. Sci 1997; 86(8): 885-890.

### Summary of the Invention

The present invention is based upon the finding of improved methods for preparing compositions in the form of solid dispersions, especially pharmaceutical formulations. It is based upon the finding that supercritical fluids, and particularly supercritical carbon dioxide, can be used as a solvent for both substances of limited solubility in aqueous solution and soluble carriers or excipients. Once a supercritical solution is formed having all of the components of the compositions, solvent may be removed and solid particles formed by rapidly expanding the solution at reduced pressure. This process produces a uniform composition with highly desirable solubility characteristics.

In its first aspect, the invention is directed to a method for preparing compositions in the form of a solid dispersion. This is achieved by first preparing a supercritical solution comprising (i) one or more insoluble or relatively insoluble substances, preferably pharmaceutically active substances, together with (ii) one or more water-soluble substances, preferably pharmaceutically acceptable carriers and (iii) these substances are dissolved in a supercritical fluid solvent such as supercritical carbon dioxide. In order to precipitate out particles, solvent is removed by rapidly expanding the supercritical solution. The term "insoluble substance" means that essentially none (≤1%) of the substance is dissolved in aqueous solution. A "relatively insoluble substance" refers to a substance that dissolves very slowly, which means that ≤10% of the substance is dissolved in aqueous solution. Typically, insoluble and relatively insoluble substances will be either neutral or in the form of a base and not in the form of a salt. Expansion to remove solvent should occur as quickly as possible. For example, essentially instantaneous expansion can be achieved by passing a supercritical solution through a small diameter nozzle or capillary tube. Although preparations can be made more slowly, this will tend to produce particles of larger diameter which therefore have less desirable solubility characteristics. Thus, the term "rapid expansion" refers to a process that occurs in less than 10 seconds and, preferably, in less than 1 second.

Preferably, the substances used in supercritical solutions are pharmaceutically active substances, preferably local anesthetics, particularly preferred are local anesthetics of amide type e.g lidocaine, prilocaine, ropivacaine, bupivacaine, mepivacaine, etidocaine and the enantiomers thereof. Supercritical solutions with lidocaine and ropivacaine are especially desirable. Any water-soluble substance may be used, preferably carriers such as polyethylene glycols. The term "water-soluble" means that essentially complete dissolution of a compound in aqueous solution can be achieved rapidly, e.g. within a period of a minute. The most preferred supercritical solution for use in the method described above contains 20-80% lidocaine and 20-80% polyethylene glycol on a weight to weight basis. Typically, rapid expansion of supercritical solution will occur at a temperature of between 25 and 100°C and at a pressure of between 10342 and 517510 kPa (1500 and 7500 psi). In addition to including these methods, the present invention also encompasses the solid dispersions, which they produce.

In a second aspect, the present invention is directed to a method of preparing a composition in the form of a solid dispersion, preferably a pharmaceutical formulation, which is similar to the first. Again, a solid dispersion is formed from a supercritical solution that uses a supercritical fluid as solvent. This solution contains (i) one or more insoluble substances and (ii) one or more relatively insoluble substances, preferably all are pharmaceutically active substances. Preferably, all effective at treating the same medical condition. Again, solvent is removed by rapid expansion of the supercritical solution to form the solid dispersion. The main advantage of pharmaceutical formulations formed in this manner are that they provide for the sequential delivery of two or more, usually related, pharamceutically active substances. Preferably, these substances are both local anesthetics, particularly preferred are local anesthetics of amide type e.g lidocaine, prilocaine, ropivacaine, bupivacaine, mepivacaine, etidocaine and the enantiomers thereof. The most preferred solid dispersion containing ropivacaine as the insoluble substance and lidocaine as the soluble substance. In general, the lidocaine should be present in formulation at a 60-90 W/W% and the ropivacaine at a 10-40 W/W%. As in the method discussed above, the rapid expansion of supercritical solution may be accomplished at a temperature of 25-100°C and at a pressure of 10342 and 51710 kPa (1500-7500 psi). The most preferred supercritical fluid solvent is supercritical carbon dioxide and the invention again includes all of the solid dispersions made using this method.

### Detailed Description of the Invention

As a nonpolar substance with zero dipole moment, carbon dioxide would not be expected to be a common solvent for relatively insoluble or insoluble substances and also for water-soluble substances. However, it has been found that SCCO₂ can function as a common solvent for such dissimilar substances as, for example, lidocaine and polyethylene glycol (PEG), especially PEG 8000. Therefore, standard procedures of RESS processing can be used to form small particles of solid dispersions of these substances with SCCO₂ as the common solvent. Enhancement of dissolution of for example pharmaceuticals from the solid dispersions occurs because of the hydrophilicity of the water-soluble substances which functions to increase the effective surface area of the insoluble or relatively insoluble substances.

In addition, it has been found that RESS processing can increase the amorphous character of suitable water-soluble substances, like carriers, especially polyethylene glycols, preferably PEG 3500, PEG 6000, PEG 8000 or those with higher molecular weight, most particlarly preferably PEG 8000, thereby further enhancing dissolution rates of the insoluble or relatively insoluble substances. Therefore, unlike solid dispersions prepared by other methods in which the optimal release of a pharmaceutically active substance only occurs at low concentrations of active substance in the formulations (e.g., less than 5% W/W pharmaceutical), the solid dispersions of the present invention prepared by RESS have enhanced rates of release at both low and high concentrations of active substances. As such, the usual limitation that solid dispersions can only be used for therapeutically potent pharmaceuticals, *i*.*e*., low pharmaceutical concentrations, does not apply to solid dispersions prepared as described herein.

It is believed that supercritical nitrous oxide may be used in the place of SCCO₂ for most formulations. These gases can be commercially purchased in a highly purified form and then converted into a supercritical state by simply pressurizing them using standard laboratory equipment. The other procedures for RESS are well known in the art (see *e.g. J. Crystal Growth 198*/*199:*767-772 (1999); *Ind Eng. Chem. Res. 34*:4987-4991 (1995); *Manuf Chem. 66*:23-25 (1995); *Biotech. Bioeng. 41*:341-346 (1993); and *Intr. J. Pharm. 152*:99-110 (1997)).

In another embodiment, the general insoluble substance/soluble substance concept can be applied to compositions comprising of (i) one or more insoluble substances and (ii) one or more relatively insoluble substances, preferably all these substances are pharmaceutically active substances. In this case, the relatively insoluble substances function as the "soluble" substances. RESS-generated formulations of this type may preferably be used to obtain the controlled release of two pharmeceutically active substances, *e.g*., two local anesthetics, with a fast initial release of the relatively insoluble substance (e.g., lidocaine) and a sustained release of the insoluble substance (*e.g*., ropivacaine). With respect to local anesthetics, the lidocaine-ropivacaine mixture, especially at 80% W/W lidocaine, is the most optimal formulation with the release of both lidocaine and ropivacaine being substantially enhanced.

The formation of small particles of a solid dispersion comprised of both a relatively insoluble and an insoluble pharmaceutically active substance with essentially the same therapeutic action may also be used with active substances having another type of medical action. In each case, the more rapidly soluble component provides for the initial release of the active substance and, by so doing, increases the effective surface area of the more soluble substance, thereby facilitating its dissolution. The physico-chemical properties of the active substance, i.e., their solubility and the influence of temperature and pressure on solubility, are important parameters to control with respect to the precipitation of substances during RESS processing. In general, the substances used should be in their neutral or base form to enhance sustained release. For example, the neutral forms of local anesthetics are released *in vitro* and *in vivo* over a longer period of time than their corresponding salt forms. The solvents used for RESS processing are, again, supercritical fluids with the most preferred being SCCO₂.

The solid dispersions made according to the present invention include also semi-solid dispersions and may take the form of smaller particles, including nanoparticles, eutectics, coprecipitated crystals, solid solutions; coprecipitated amorphous systems: and/or coprecipitated glasses and glass solutions. The dispersions may be administered by any route compatible with the particular condition being treated. For example, compositions may be given orally, rectally, by inhalation, topically, parenterally, vaginally, sublingually, or bucally.

Having now described the invention, the same will be more readily understood through reference to the following Examples, which are provided by way of illustration

### Examples

### Example 1: Pharmaceutical Solid Dispersions

Solid dispersions of lidocaine/PEG 8000 and lidocaine/ropivacaine were prepared by mixing appropriate amounts of each component and loading them individually into sample cartridges of standard RESS instrumentation. The sample mixtures were equilibrated with SCCO₂ before particle formation. Solid dispersions were obtained for the following formulations:

**Table 1: Formulations Prepared by RESS**

| Formulation No. | Lidocaine Base (W/W%) | PEG 8000 (W/W%) | Ropivacaine (W/W%) |
|---|---|---|---|
| #1 | 20 | 80 | - |
| #2 | 30 | 70 | - |
| #3 | 40 | 60 | - |
| #4 | 70 | 30 | - |
| #5 | 80 | 20 | - |
| #6 | 60 | - | 40 |
| #7 | 70 | - | 30 |
| #8 | 80 | - | 20 |
| #9 | 90 | - | 10 |

Differential scanning calorimetry (DSC) of general standard was used for analysis. The disolution rate of the active substance, lidocaine, from the solid dispersion with PEG 8000 was measured as % release of lidocaine.

### Example 2: Release Rate of Lidocaine/Ropivacaine Solid Dispersions

The objective of this example was to examine the release of local anesthetic agent in a lidocaine/ropivacaine formulation. Since ropivacaine is about 50 times more therapeutically potent than lidocaine, therapeutic effect vs. time profiles were constructed to predict the overall therapeutic effect of the formulation. In these plots, the local anesthetic effect of ropivacaine was normalized to the effect of lidocaine by multiplying by a factor of 50 and the therapeutic effect of the mixture is calculated. *i.e.*, simulated, by the sum of the effect of lidocaine and the normalized effect of ropivacaine. For example, a total dose of 100 mg of the 80% lidocaine/ropivacaine mixture would be equivalent to (80+(20x50)) = 1080 mg equivalents of lidocaine. A resulting total percentage release curve was constructed and therapeutic effects were plotted as a function of time. It was found that this formulation achieved continuous release of total anesthetic for 8 hours or more, following an initial burst of release. Similar results were found for the 20% and 50% W/W mixtures, *i.e.*, both of these latter formulations also achieved sustained release for more than 8 hours.

## Claims

1. A method of preparing a solid dispersion comprising:
(a) preparing a supercritical solution comprising:
(i) one or more insoluble or relatively insoluble substances;
(ii) one or more water-soluble substances; and
(iii) a supercritical fluid that serves as solvent and is capable of dissolving said substances defined in (i) and (ii);
(b) removing solvent by rapid expansion of said supercritical solution to form said solid dispersion.

2. A method according to clam 1, wherein one of the substances defined in (i) is an insoluble substance.

3. A method according to any one of claims 1-2, wherein the substances defined in (i) is pharmaceutically active substances.

4. A method according to claim 3, wherein said pharmaceutically active substances are local anesthetics.

5. A method according to claim 4, wherein said local anesthetics are local anesthetics of amide type.

6. A method according to claim 5, wherein said local anesthetics are selected from the group consisting of lidocaine, prilocaine, ropivacaine, bupivacaine, mepivacaine, etidocaine and the enatiomers thereof.

7. A method according to claim 1, wherein the water-soluble substances are carriers, preferably pharmaceutically acceptable carriers.

8. A method according to claim 7, wherein the water-soluble carriers are polyethylene glycols.

9. A method according to claim 8, wherein the polyethylene glycols are PEG 3500, PEG 6000, PEG 8000 or those with higher molecular weight, preferably PEG 8000.

10. A method according to any one of claims 1-9, wherein said supercritical solution comprises 20-80 W/W% lidocaine and 20-80 W/W% polyethylene glycol.

11. A method according to any one of claims 1-10. wherein said supercritical fluid solvent is supercritical carbon dioxide.

12. A method according to any one of claims 1-11, wherein said rapid expansion of supercritical solution occurs at a temperature of 25-100°C and at a pressure of 10342 - 51710 kPa (1500-7500 psi).

13. A solid dispersion obtainable by the method of any one of claims 1-12.

14. A method of preparing a solid dispersion comprising:
(a) preparing a supercritical solution comprising:
(i) one or more insoluble substances;
(ii) one or more relatively insoluble substances; and
(iii) a supercritical fluid that serves as solvent and is capable of dissolving said substances defined in (i) and (ii);
(b) removing solvent by rapid expansion of said supercritical solution to form said solid dispersion.

15. A method according to claim 14, wherein the substances defined in (i) and (ii) are pharmaceutically active substances.

16. A method according to claim 15, wherein said pharmaceutically active substances are local anesthetics.

17. A method according to claim 16, wherein said local anesthetics are local anesthetics of amide type.

18. A method according to claim 17, wherein said local anesthetics are selected from the group consisting of lidocaine, prilocaine, ropivacaine, bupivacaine, mepivacaine, etidocaine and the enatiomers thereof.

19. A method according to claim 17, wherein the substance defined in (i) is ropivacaine and the substance defined in (ii) is lidocaine.

20. A method according to claim 19, wherein said supercritical solution comprises 60-90 W/W% lidocaine and 10-40 W/W% ropivacaine.

21. A method according to any one of claims 14-20, wherein said supercritical solution is supercritical carbon dioxide.

22. A method according to any one of claims 14-21, wherein said expansion of supercritical solution occurs at a temperature of 25-100°C and at a pressure of 1500-7500 psi.

23. A solid dispersion obtainable by the method of any one of claims 14-22.

24. A solid pharmaceutical formulation in the form of a solid dispersion according to claim 23, said formulation having a fast initial release of one or more substances defined in (i) and a sustained release of one or more substances defined in (ii).

25. A solid pharmaceutical formulation according to claim 24, wherein the substance defined in (i) is ropivacaine and the substance defined in (ii) is lidocaine.

## Patentansprüche

1. Verfahren zur Herstellung einer Feststoffdispersion, umfassend:
(a) das Herstellen einer überkritischen Lösung, umfassend:
(i) eine oder mehrere unlösliche oder relativ unlösliche Substanzen;
(ii) eine oder mehrere wasserlösliche Substanzen und
(iii) ein überkritisches Fluid, das als Lösungsmittel dient und befähigt ist, die in (i) und (ii) definierten Substanzen zu lösen,
(b) das Entfernen des Lösungsmittels durch rasche Expansion der überkritischen Lösung unter Bildung der Feststoffdispersion.

2. Verfahren gemäß Anspruch 1, wobei eine der in (i) definierten Substanzen eine unlösliche Substanz ist.

3. Verfahren gemäß einem der Ansprüche 1-2, wobei die in (i) definierten Substanzen pharmazeutisch wirksame Substanzen sind.

4. Verfahren gemäß Anspruch 3, wobei die pharmazeutisch wirksamen Substanzen Lokalanästhetika sind.

5. Verfahren gemäß Anspruch 4, wobei die Lokalanästhetika Lokalanästhetika vom Amid-Typ sind.

6. Verfahren gemäß Anspruch 5, wobei die Lokalanästhetika aus der Gruppe, bestehend aus Lidocain, Prilocain, Ropivacain, Bupivacain, Mepivacain, Etidocain und den Enatiomeren davon, ausgewählt sind.

7. Verfahren gemäß Anspruch 1, wobei die wasserlöslichen Substanzen Träger, vorzugsweise pharmazeutisch verträgliche Träger, sind.

8. Verfahren gemäß Anspruch 7, wobei die wasserlöslichen Träger Polyethylenglykole sind.

9. Verfahren gemäß Anspruch 8, wobei die Polyethylenglykole PEG 3500, PEG 6000, PEG 8000 oder solche mit höherem Molekulargewicht, vorzugsweise PEG 8000, sind.

10. Verfahren gemäß einem der Ansprüche 1-9, wobei die überkritische Lösung 20-80 W/W% Lidocain und 20-80 W/W% Polyethylenglykol umfaßt.

11. Verfahren gemäß einem der Ansprüche 1-10, wobei das überkritische Fluidlösungsmittel überkritisches Kohlendioxid ist.

12. Verfahren gemäß einem der Ansprüche 1-11, wobei die rasche Expansion der überkritischen Lösung bei einer Temperatur von 25-100°C und bei einem Druck von 10342-51710 kPa (1500-7500 psi) erfolgt.

13. Feststoffdispersion, erhältlich durch das Verfahren gemäß einem der Ansprüche 1-12.

14. Verfahren zur Herstellung einer Feststoffdispersion, umfassend:
(a) das Herstellen einer überkritischen Lösung, umfassend:
(i) eine oder mehrere unlösliche Substanzen,
(ii) eine oder mehrere relativ unlösliche Substanzen und
(iii) ein überkritisches Fluid, das als Lösungsmittel dient und befähigt ist, die in (i) und (ii) definierten Substanzen zu lösen,
(b) das Entfernen des Lösungsmittels durch rasche Expansion der überkritischen Lösung unter Bildung der Feststoffdispersion.

15. Verfahren gemäß Anspruch 14, wobei die in (i) und (ii) definierten Substanzen pharmazeutisch wirksame Substanzen sind.

16. Verfahren gemäß Anspruch 15, wobei die pharmazeutisch wirksamen Substanzen Lokalanästhetika sind.

17. Verfahren gemäß Anspruch 16, wobei die Lokalanästhetika Lokalanästhetika vom Amid-Typ sind.

18. Verfahren gemäß Anspruch 17, wobei die Lokalanästhetika aus der Gruppe, bestehend aus Lidocain, Pruilocain, Ropivacain, Bupivacain, Mepivacain, Etidocain und den Enatiomeren davon, ausgewählt sind.

19. Verfahren gemäß Anspruch 17, wobei die in (i) definierte Substanz Ropivacain ist und die in (ii) definierte Substanz Lidocain ist.

20. Verfahren gemäß Anspruch 19, wobei die überkritische Lösung 60-90 W/W% Lidocain und 10-40 W/W% Ropivacain umfaßt.

21. Verfahren gemäß einem der Ansprüche 14-20, wobei die überkritische Lösung überkritisches Kohlendioxid ist.

22. Verfahren gemäß einem der Ansprüche 14-21, wobei die Expansion der überkritischen Lösung bei einer Temperatur von 25-100°C und bei einem Druck von 1500-7500 psi erfolgt.

23. Feststoffdispersion, erhältlich durch das Verfahren gemäß einem der Ansprüche 14-22.

24. Pharmazeutische Feststoffformulierung in der Form einer Feststoffdispersion gemäß Anspruch 23, wobei die Formulierung eine schnelle anfängliche Freisetzung von einer oder mehreren, in (i) definierten Substanzen und eine verzögerte Freisetzung von einer oder mehreren, in (ii) definierten Substanzen aufweist.

25. Pharmazeutische Feststoffformulierung gemäß Anspruch 24, wobei die in (i) definierte Substanz Ropivacain ist und die in (ii) definierte Substanz Lidocain ist.

## Revendications

1. Procédé de préparation d'une dispersion solide, comprenant:
(a) la préparation d'une solution supercritique comprenant:
(i) une ou plusieurs substances insolubles ou relativement insolubles;
(ii) une ou plusieurs substances hydrosolubles; et
(iii) un fluide supercritique qui sert de solvant et qui est capable de dissoudre lesdites substances définies dans (i) et (ii);
(b) l'élimination du solvant par dilatation rapide de ladite solution supercritique pour former ladite dispersion solide.

2. Procédé selon la revendication 1, dans lequel l'une des substances définies dans (i) est une substance insoluble.

3. Procédé selon l'une quelconque des revendications 1 et 2, dans lequel les substances définies dans (i) sont des substances actives sur le plan pharmaceutique.

4. Procédé selon la revendication 3, dans lequel lesdites substances actives sur le plan pharmaceutique sont des anesthésiques locaux.

5. Procédé selon la revendication 4, dans lequel lesdits anesthésiques locaux sont des anesthésiques locaux de type amide.

6. Procédé selon la revendication 5, dans lequel lesdits anesthésiques locaux sont choisis dans le groupe constitué par la lidocaïne, la prilocaïne, la ropivacaïne, la bupivacaïne, la mépivacaïne, l'étidocaïne et leurs énantiomères.

7. Procédé selon la revendication 1, dans lequel les substances hydrosolubles sont des véhicules, de préférence des véhicules acceptables sur le plan pharmaceutique.

8. Procédé selon la revendication 7, dans lequel les véhicules hydrosolubles sont des polyéthylèneglycols.

9. Procédé selon la revendication 8, dans lequel les polyéthylèneglycols sont les PEG 3500, PEG 6000, PEG 8000 et ceux ayant un poids moléculaire plus élevé, de préférence le PEG 8000.

10. Procédé selon l'une quelconque des revendications 1 à 9, dans lequel ladite solution supercritique comprend 20 à 80% P/P de lidocaïne et 20 à 80% P/P de polyéthylèneglycol.

11. Procédé selon l'une quelconque des revendications 1 à 10, dans lequel ledit solvant fluide supercritique est un dioxyde de carbone supercritique.

12. Procédé selon l'une quelconque des revendications 1 à 11, dans lequel ladite dilatation rapide de la solution supercritique a lieu à une température de 25-100°C et à une pression de 10342 à 51710 kPa (1500 à 7500 psi).

13. Dispersion solide pouvant être obtenue par le procédé selon l'une quelconque des revendications 1 à 12.

14. Procédé de préparation d'une dispersion solide, comprenant:
(a) la préparation d'une solution supercritique comprenant:
(i) une ou plusieurs substances insolubles;
(ii) une ou plusieurs substances relativement insolubles; et
(iii) un fluide supercritique qui sert de solvant et qui est capable de dissoudre lesdites substances définies dans (i) et (ii);
(b) l'élimination du solvant par dilatation rapide de ladite solution supercritique pour former ladite dispersion solide.

15. Procédé selon la revendication 14, dans lequel les substances définies dans (i) et (ii) sont des substances actives sur le plan pharmaceutique.

16. Procédé selon la revendication 15, dans lequel lesdites substances actives sur le plan pharmaceutique sont des anesthésiques locaux.

17. Procédé selon la revendication 16, dans lequel lesdits anesthésiques locaux sont des anesthésiques locaux de type amide.

18. Procédé selon la revendication 17, dans lequel lesdits anesthésiques locaux sont choisis dans le groupe constitué par la lidocaïne, la prilocaïne, la ropivacaïne, la bupivacaïne, la mépivacaïne, l'étidocaïne et leurs énantiomères.

19. Procédé selon la revendication 17, dans lequel la susbtance définie dans (i) est la ropivacaïne et la substance définie dans (ii) est la lidocaïne.

20. Procédé selon la revendication 19, dans lequel ladite solution supercritique comprend 60 à 90% P/P de lidocaïne et 10 à 40% P/P de ropivacaïne.

21. Procédé selon l'une quelconque des revendications 14 à 20, dans lequel ladite solution supercritique est du dioxyde de carbone supercritique.

22. Procédé selon l'une quelconque des revendications 14 à 21, dans lequel ladite dilatation de la solution supercritique a lieu à une température de 25-100°C et à une pression de 1500 à 7500 psi.

23. Dispersion solide pouvant être obtenue par le procédé selon l'une quelconque des revendications 14 à 22.

24. Formulation pharmaceutique solide sous forme d'une dispersion solide selon la revendication 23, ladite formulation ayant une libération initiale rapide d'une ou de plusieurs substances définies dans (i) et une libération prolongée d'une ou de plusieurs substances définies dans (ii).

25. Formulation pharmaceutique solide selon la revendication 24, dans laquelle la substance définie dans (i) est la ropivacaïne et la substance définie dans (ii) est la lidocaïne.
